# EUROPEAN PATENT SPECIFICATION

(11) **EP 1 646 410 B1**
(45) Date of publication and mention of the grant of the patent: **11.11.2009**
(21) Application number: 04743979.9
(22) Date of filing: 19.07.2004
(51) Int. Cl.: A61L 17/10, A61F 2/02

(54) **SYNTHETIC, BIOABSORBABLE POLYMER MATERIALS AND IMPLANTS**
SYNTHETISCHE, BIOABSORBIERBARE POLYMER-MATERIALIEN UND IMPLANTATE
MATERIAUX ET IMPLANTS POLYMERES BIOABSORBABLES SYNTHETIQUES

(30) Priority: 17.07.2003 FI 20031090; 15.06.2004 FI 20045223
(43) Date of publication of application: 19.04.2006
(73) Proprietor: BIORETEC OY, 33720 Tampere (FI)
(72) Inventor: SUOKAS, Esa, FIN-33580 Tampere (FI); ASHAMMAKHI, Nureddin, Staffordshire ST4 6BY (GB); TÖRMÄLÄ, Pertti, FIN-33300 Tampere (FI)
(74) Representative: Hakola, Unto Tapani
(86) International application number: PCT/IB2004/002318
(87) International publication number: WO 2005/007209

(56) References cited:
- EP-A1- 1 157 708
- US-B1- 6 406 498
- PIRKKA MAKELA ET AL.: 'Strength retention properties of self-reinforced poly L-lactide (SR-PLLA) sutures compared with polygyconate (MaxonR) and polydioxanone (PDS) sutures. An in vitro study' BIOMATERIALS vol. 23, no. 12, 2002, pages 2587 - 2592, XP004348032
- BLEACH N.C. ET AL.: 'Effect of filter content on mechanical and dynamic mechanical properties of particulate biphasic calcium phosphate-polylactide composites' BIOMATERIALS vol. 23, no. 7, 2002, pages 1579 - 1585, XP004335631

## Description

### FIELD OF THE INVENTION

The invention relates to synthetic, bioabsorbable polymer materials and implants, like fibres, sutures, meshes and other tissue management, wound closure or tissue engineering devices.

### BACKGROUND OF THE INVENTION

Wounds (soft or hard tissue) are basically classified from the surgical point of view into:

| | |
|---|---|
| a. | clean |
| b. | clean contaminated |
| c. | contaminated |
| d. | dirty |
| e. | infected |

Antibiotic administration is indicated as therapeutic measure for established infection and as prophylactic measure in cases where infection is anticipated, expected or is a potential risk.

Surgery, in general, how careful surgeon ever is, leads to some degree of devitalization and temporary de-vascularization, with various degrees of haematoma formation. These factors combined with the metabolic response to surgery or injury, with suppressed immunity in surgical or trauma patients, etc, provide a good basis for development of infection in the surgical wounds. In addition, along with the increased use of implants (sutures being the commonest), susceptibility to infection is increased due also to a combination of factors, e.g. reduced dose of infective bacteria that is needed otherwise to cause infection in healthy patients, susceptibility of implants to bacterial attachment, etc. Bacterial attachment in many of the common infection causative bacteria are associated with biofilm formation which occurs very early in the onset of events in the evolution of infection. Biofilm has probably evolved as a collaborative community of bacteria that try to enhance chances of their survival. Hence, planktonic bacteria once attached, most of them produces glycocalyx and a community of bacteria gets surrounded with this protective structure. Macrophages can not affect the bacteria, rather they are frustrated and their released cytokines may further complicate local tissue injury. Antibodies can not get access to bacteria hiding under the glycocalyx either. Antibiotic may be needed in manifold doses to produce their antibacterial effect as they would do with planktonic bacteria. In addition, antibiotic activity suffers due to many factors. While it is mandatory to treat infection, the presence of implant and biofilm renders the therapy ineffective without its removal, a procedure that must be undertaken to eradicate such a surgical infection. This leads to consumption of time and costs as well as raising the risks of morbidity and mortality in surgical and trauma patients. It would be wise accordingly, rather to prevent bacteria from attachment and glycocalyx formation from the start.

With the invention of synthetic sutures (Dexon^{R}) in 1969 and its wide clinical acceptance, Vicryl^{R} followed in 1974, and both gained wide acceptance and are in daily clinical use. Others, such as Maxon^{R} and PDS have also been developed. Most of developments focused on manipulating the initial strength and strength retention properties, handling properties, etc. Monofilamentous sutures have been long considered to be associated with lower rate of infection as compared to multifilamentous ones. Explanations for this observation, such as capillary effect, ductility etc, have been presented in the literature.

Knot characteristics (holding) is however, better with multifilamentous, but it has been always the responsibility of surgeon to balance risks and benefits and make the right decision with the choices made, including the type of suture selected for each defined procedure.

Surgical devices prepared from extruded materials include mesh prostheses conventionally used to repair hernias. Such mesh fabric prostheses are also used in other surgical procedures, including the repair of anatomical defects of the abdominal wall, diaphragm, and body walls, correction of defects in the genitourinary system, and repair of traumatically damaged organs such as the spleen, liver or kidney or in inducing the formation of fibrous tissue small joint in fingers of rheumatoid patients (U.S. Pat. No. 6,113,640) or as scaffolds for tissue engineering (Gaissmaer et al. 2002, Länsman et al. 2002). Mesh fabrics for use in connection with hernia repairs are disclosed in U.S. Patent Nos. 5,292,328; 4,769,038 and 2,671,444. Knitted and woven fabrics constructed from a variety of synthetic fibers and the use of the fabrics in surgical repair are also discussed in U.S. Patent Nos. 3,054,406; 3,124,136; 4,193,137; 4,347,847; 4,452,245; 4,520,821; 4,633,873; 4,652,264; 4,655,221; 4,838,884; 5,002,551; and European Patent No. 334,046.

Surgical devices prepared from extruded filaments also include mono-and multi-filament sutures (e.g. Kangas et al. J Biomed Mater Res. 2001, 58(1): 121-6, Mäkelä et al. Biomaterials 2002, 23(12): 2587-92, US Pat 4,557,264, 4,911,165, U.S. Patents 6,350,284 and 6,398,814).

US patent No. 4,853,225 describes a method of combating infection in patients, where a medicament depot is implanted in the patient. The medicament depot consists of a physiologically acceptable excipient, which achieves a delayed release.

Polymeric drug delivery implants for treatment of infection of the bone have also been developed, as is disclosed for example in US Patent No. 5,281,419 showing a fracture-fixation device containing an antibiotic in a biodegradable polymer carrier.

It is important during the healing process and subsequent thereto that the surgical devices placed within the body do have pharmacological action in addition to their primary function as tissue management devices. Most importantly, such devices should resist the attachment and growth of bacteria, as well as biofilm formation on or immediately about them. The other important function of surgical devices can be the function of releasing analgesic drugs to provide analgesia to the patient postoperatively. Devices that utilize anti-microbial agents applied to their surfaces such as sutures coated with germicidal ions are disclosed in US Pat. 3,642,003 and 3,862,304. Devices, e.g. sutures, comprising coatings of antimicrobial agents are disclosed in US Pat. 5,019,096. Substrates made from filaments, which substrates then are impregnated with an anti-microbial agent, are disclosed in US Pat 5,534,288. The antimicrobial agent is said to flow into the interstices between the filaments from which the substrate is formed. It can be more advantageous if multifunctional surgical devices, used for either soft or hard tissue management devices, e.g. as sutures, mesh, etc., prepared from materials that could be made to contain pharmacological agents such as, but not limited to, anti-bacterial, analgesic, or anti-inflammatory agents without coating and/or impregnation processes such as those discussed in the patents above.

Surgical devices having antimicrobial activity would have many potential uses. One example can be the use in abdominal surgery and trauma management where commensals may contaminate the wound. Lowered rate of infection, more invasive and early treatment would be contemplated by surgeons, such as in cases of trauma. Such a policy would be otherwise impossible with the current types of suture available to surgeons. One could think of early intervention to undertake operative rather than conservative management because of more confidence in the type of implant used. This will consequently lead to increased number of cases that can be managed using this novel implant. More lives can be saved and complications reduced.

In one example, soft tissue wounds with delayed-presentation, where the potential of infection is increased, though infection can not be established as a diagnosis at the time of presentation, can be managed more effectively and appropriately. In cases where vascularity is compromised such as in the increasing number of patients suffering from peripheral vascular diseases, the importance of raised local concentration of antibiotic, just to where it should be and the time has to be there, will have a clear benefit using this type of implant, the subject of the current invention. Using a suture with antibacterial properties will increase chances of the body to prevent bacteria from developing from a status of contamination into a status of infection.

In one example, amputation, due to traumatic or vascular reasons, the susceptibility to infection can be reduced by fighting bacteria locally where antibiotics administered systemically may not always achieve sufficient concentrations in local tissue environment with compromised vascularity.

In one example, the operations of cholesystectomy, GI anastomoses, and urology can be performed with increased safety.

Previously-made sutures such as the commercially available Maxon® and PDS®, although have good strength properties, when they were tested for their tensile strength retention in comparison to sutures that we have made of PLDLA and PLLA, more prolonged strength retention was seen with PLDLA or PLLA (Mäkelä et al. Biomaterials 2002, 23(12): 2587-92 and Kangas et al. J Biomed Mater Res. 2001, 58(1): 121-6). These offer more secure and ensured support and reduced risk of failure especially in mechanically demanding repair where the wound is exposed to continuous distraction forces such as that of tendons, ligaments, or abdominal wall, e.g. hernial mesh repair or following trauma/infection (Kangas et al. J Biomed Mater Res. 2001, 58(1): 121-6).

However, without antibacterial properties, surfaces of polymers remain prone to bacterial attachment and biofilm formation that is difficult to eradicate.

EP 1157708 A2 discloses bioabsorbable or non-bioabsorbable materials, e.g. polypropylene, that contain antimicrobial materials homogeneously dispersed in a polymeric matrix. The examples of this publication relate to polypropylene and the antimicrobial agents are specific synthetic diphenyl ethers.

### SUMMARY OF THE INVENTION

In our recent work with ciprofloxacin-releasing PLGA 80/20 granules, we have found that bacterial attachment and biofilm formation is reduced with these materials as compared with control materials (not containing ciprofloxacin or titanium). Ciprofloxacin screws were associated with clear inhibition area of about 30 mm diameter, when incubated in agar plates containing S. epidermides, as compared to 0 mm with controls.

The present invention discloses novel, synthetic bioabsorbable multifunctional surgical devices of new physical structure that are appropriate for use to mend, repair or treat wounds that are contaminated, infected or at increased risk of infection, due whatever reason, or where infection consequences would be disastrous, should infection supervene. The multifunctional implant of this invention comprises: 1) a synthetic bioabsorbable polymeric (polymer, copolymer or polymer alloy) carrier into which is mixed ("dispersed") 2) a pharmacological agent, for example an antibiotic or antibiotic mixture effective for the prevention and/or healing of infection, such as in infected wounds or wounds with potential risk of infection or should infection occur, with consequences that would be catastrophic, such as trauma wounds and wounds breaching natural barriers of heavily-contaminated environment, such as the colon; and optionally 3) an additive/coating that can facilitate the function and handling of the material as suture, e.g. to improve knot making and knot holding or its biocompatibility or its visibility, e.g. quinone. The multifunctional device of the present invention can be in any appropriate form into which the polymer matrix, including antibiotic(s) can be formed with the polymer technological processing methods, which contain: a) a bioabsorbable polymer, copolymer or polymer alloy ("polymeric") matrix; b) the pharmacological agent, such as an antibiotic (antibacterial) and/or anti-inflammatory and/or healing promoting agent or any mixture thereof, dispersed in the matrix, and c) optionally an additive agent to facilitate device recognition and/or handling. To put it more precisely, the device according to the present invention is primarily characterised in that it has oriented structure where mixing-orientation induces the production of oval and/or spindle-shaped cavities resulting from initially rounded holes resulting from the inclusion of the particles of the pharmacological agent. According to an advantageous embodiment of the present invention the device is primarily characterised in that it comprises:
- a synthetic bioabsorbable polymeric matrix, and
- an additive phase formed of one or more pharmacological agents dispersed into said polymeric matrix,
wherein the device has reduced modulus and increased elasticity.

In particular, the multifunctional devices of the present invention can be used for: 1) approximation of wound edges (wound closure) to promote healing; and 2) releasing antibiotic to prevent bacterial attachment, proliferation and biofilm formation and consequently prevent infection.

An advantage of the multifunctional bioabsorbable devices of the present invention is that they can be used in the management of wounds (including fractures) and incisions (including osteotomies) in patients who have a high risk of developing postoperative infections, such as immunocompromised patients weather due to congenital or acquired reasons. Examples include diabetic patients, patients on dialysis, patients on steroids. Patients with compromised circulation either due to trauma or disease may also very well benefit from using the devices of the present invention in combating against infection or at least bringing the risk down, which would be otherwise impossible to achieve with conventional corresponding devices, without using extra measures such as the administration of antibiotics as a separate procedure or measure.

Biostable (non-bioabsorbable) materials entail their permanent residence in tissues or need for removal. This is automatically associated with other risks such as that of extrusion, migration, etc and problems which are known in the field of implantology. The use of bioabsorbable sutures may not always provide sufficient support for required prolonged periods of time. A bioabsorbable filament that combines the properties of bioabsorbable materials (eliminated from the body) and non-bioabsorbable materials (prolonged support) may be obtained by using the patented manufacturing technique of SR (self-reinforcing (US Pat. No. 6,406,498), such as demonstrated in our experiment (Mäkelä et al. Biomaterials 2002, 23(12): 2587-92). SR-PLDLA monofilaments were found to retain their strength more than Maxon® and PDS®. Compounding PLDLA 70/30 material with ciprofloxacin was found to significantly reduce bacterial attachment, growth and biofilm formation on their surface (See example 1 hereinbelow).

Provided herein are also methods for forming the multifunctional bioabsorbable devices of the present invention and methods of using the same.

### DESCRIPTION OF THE DRAWINGS

In the following, the present invention will be described in more detail with reference to the figures, in which
Fig. 1 is a schematic illustration of the structure of the device of the present invention following mixing and following orientation,
Fig. 2 is a schematic illustration of the cavitation effect of the drawing procedure,
Fig. 3 is a schematic illustration of the mesh structure of the device of the present invention, and
Fig. 4 is a schematic illustration of the braid structure of the device of the present invention.

### DETAILED DESCRIPTION OF THE INVENTION

Multifunctional devices and implants of the present invention are fabricated to comprise: 1) a synthetic bioabsorbable polymeric (a polymer, a copolymer or polymer alloy) matrix; 2) an antibiotic phase (preferably 1 to 20 w/w%) dispersed into the polymeric matrix, and 3) optionally an additive to facilitate the function or handling properties or outcome of use such as coating with wax, using a quinone dye to render it visible in the blood and tissues (surgical field), growth factors to accelerate healing, or anticancer drugs when used in cancer patients, or the like.

In the preferred embodiment of the present invention, the devices should have a surface that is not favourable for bacteria, yet does not interfere with wound healing to the extent that affects clinical outcome adversely.

The multifunctional devices of the present invention can be made in any appropriate form to contain a polymer matrix and antibiotic(s), employing polymer technological processing methods. Typical forms are mono- and/or multifilamentous sutures and their derivatives such as meshes and scaffolds.

The bioabsorbable polymeric matrix of the multifunctional devices of the present invention can be selected from a variety of synthetic bioabsorbable polymers, which are described extensively in the literature. Such bioabsorbale, biocompatible polymers, which may release antibiotic(s) over appropriate period of time enough to combat or reduce the risk of infection in defined areas of soft or hard tissue wounds, and which may also act as suitable matrices to include other biomolecules or pharmacological agents or to transplant cells, can include poly-α-hydroxy acids, e.g. polylactides, polyglycolides and their copolymers, polyanhydrides, polyorthoesters, segmented block copolymers of polyethylene glycol and poly terphthalate (Polyactive^{™}), tyrosine derivative polymers or poly(ester amides). Suitable bioabsorbable polymers that can be used in manufacturing of multifunctional devices of the present invention are mentioned in previous patents such as US Pat. No. 4,968,317, US Pat. No. 5,618,563, FI Pat. No. 98136, FI Pat. No. 100217B and in "Biomedical Polymers" edited by S.W. Shalaby, Carl Hanser, Verlag, Munich, Vienna, New York, 1994 and in many other references cited in the above-mentioned publications. Particular polymer that will be used for certain and defined devices will be selected depending on particular indication as governed, e.g. by risk of infection, area of application, type of tissue, vascularity local status and immunological status of the patient, etc. For example, in treating areas with poor vascularity and areas that need prolonged support such as fascial body walls, and tendons a polymer with prolonged strength retention is preferred, such as PLDLA. While when treating a wound with relatively better vascularity in the upper limb of in facial area, or in intestine a faster-to-degrade and faster-to-resorb polymer can be used such as PLGA. Hence polymers will be selected according to intended application and surgical indication.

Variations in the composition of each of the materials used for manufacturing the multifunctional devices of the present invention, such as the molecular weight of the polymer of the matrix and the relative amount of the antibiotic(s) component will affect the rate and amount of antibiotic release, and hence controlling them will allow modifications to be made to have a multifunctional device with controlled release in terms of rate and amount, that can be tailored according to various situations as mentioned above. In general, the lower is the molecular weight of the polymer used, the faster are the biodegradation and drug release.

According to the present invention, multifunctional surgical devices are prepared to have more than one function, i.e. surgical tissue management such as approximation of wound edges, or repair, or fixation of soft tissues such as the skin, muscle, tendon, etc. or hard tissues such as cartilage and/or bone, or soft tissue to hard tissue such as ligament to bone. Besides their primary function as surgical devices, they are also intended to confer extended function to the organism, for example but not limited to, combating bacterial attachment, growth and biofilm formation, in one preferred embodiment of this invention, or by supplying analgesic, anti-inflammatory or antipyretic agents in the other preferred embodiment. More than one agent can be added to render the device more efficient, more biocompatible, to have better outcome or more durable or safer function where needed, according to the indication of the implantation.

The device disclosed in the present invention has the pharmacological agent, in preferred embodiment, antibacterial agent dispersed therethrough (see examples below). The devices are multifunctional in the sense that they provide: 1) tissue management (repair or fixation or *in situ* / *in vivo* / *in vitro* tissue management/engineering) in addition to releasing pharmacological agents to make them, e.g. substantially resistant to bacterial attachment, growth and biofilm formation thereon without the need for additional surface treatment of the device, such as coating or impregnation or to the medium or otherwise. In case of infection prophylaxis, supplementary antibiotic doses, can be hence, minimized or eliminated reducing the side effects.

The pharmacological agent, such as an antibacterial agent (antibiotic) is homogeneously dispersed through the matrix in an amount effective to produce multifunctional surgical devices capable of adding a second function, in addition to their primary function of tissue management. As example, but not limited to, preferred embodiment of the present invention, the said multifunctional device can substantially prevent in *situ* attachment, growth of bacteria, and biofilm formation on the surface of such surgical devices or inducing analgesia in the patient.

Preferred are matrix materials that have already been accepted for use as tissue management devices, for both soft and hard tissues, e.g. sutures, mesh prostheses (soft tissue management or repair), plates, screws or tacks (for hard tissue management such as fixation or regeneration) or for tissue engineering, either as 2D and 3D scaffolds, e.g. U.S. Pat. 6,350,284 and 6,398,814.

Antibiotic(s) of choice used in these multifunctional devices as one component, to prevent and/or treat infection can be selected from a variety of antibiotics. Antibiotic classes that the antibiotic of choice may be selected from, comprise, e.g. aminoglycosides, quinolones or β-lactams. Examples include ciprofloxacin, gentamycin, tobramycin, erythromycin, vancomycin, oxacillin, cloxacillin, methicillin, lincomycin, ampicillin, colisin and cephalosporines which are in wide clinical use in all surgical fields. Suitable antibiotics for surgical patients can be found from the standard surgical textbooks.

A variety of biomolecules that enhance wound healing can also be added. An anti-inflammatory drug can also be added, and its particles can be included in the matrix together with the particles of an antibiotic.

Coating materials that facilitate handling or colouring dyes that facilitate the discrimination or recognition can also be included.

Several methods for manufacturing multifunctional devices of the present invention are available. Raw material [polymer(s)] and [antibiotic(s)] can be initially in the form of powder, granules, flakes, fibers or other particulate form and can be mixed together mechanically. The resulting mixure can then be heated and processed using the known methods of polymer technology. These include the use of a batch mixer (e.g. Brabender, Banburry, Farrel or Sigme mixer), continuous extrusion process using, e.g. single or twin screw extruder or special conical screw extruder and injection molding, compression molding or ultrasonic compression so that the polymeric matrix melts or softens and the antibiotic phase is dispersed into the polymeric matrix.

The pharmacological agent (antibacterial agent, anti-inflammatory agent etc.) should retain its solid particulate form in the melt-processing temperature of the matrix because of its melting or decomposition temperature higher than the melting temperature of the matrix.

In one preferred embodiment of the present invention the mixture can be spinned into fibres either using melt spinning of the melted mixture of polymer and particles, or with spinning of particles-containing polymer solution. Such fibres can be made into multifunctional devices, sutures in this case, and fibre-based fabrics. Such sutures can be used to mend, close or repair wounds of both soft and hard tissues. Such devices are of special importance because of their multifunctionality in: a) promoting healing of wounds by approximating wound edges and reducing haematoma formation and consequently the amount of scar tissue formation, b) act as prophylactic and/or therapeutic antibiotic-releasing system combating against bacterial attachment and biofilm (glycocalyx) formation, c) a third function can be added according to preferred mode of application and specific clinical indication, e.g. in releasing growth factors, anticancer, or analgesic drugs. Such multifunctional devices can be manufactured of matrix polymer(s), and antibiotic(s) and any added third agent that facilitate or adds to improve the functions of the said device, or pellets or granules made of them using polymer processing methods such as continuous compounding extrusion, injection molding compression molding, or pultrusion. Preforms made using the above-mentioned methods can also be oriented and self-reinforced by using solid-state deformation as can be achieved by drawing, shearing, compression, rolling, hydrostatic or ram extrusion. Any mechanical solid-state processing of the matrix that induce cavities around the pharmacological agent particles inside the matrix can be used.

The device, e.g. sutures or mesh, can be manufactured from bioabsorbable fibers using any of the known methods from mechanical textile and plastics technology. The thickness of the fibers can vary from about 1 micrometer to about 200 micrometers. In preferred embodiment of the invention, the fiber thickness is between ca. 5 micrometers and ca. 150 micrometers.

Structures suitable for making the multifunctional device, wherein the device is a mesh, can be, for example, a cloth, a narrow fabric, a knit, a weave, a braid, or a web. In any of such a case, the structure should be porous with pore size from ca 30 micrometers to ca 1000 micrometers, preferably between can. 50 micrometers to ca. 400 micrometers. The mesh can be manufactured using one type of fiber, for example PGA or PLA or their copolymeric fibers. It is also possible to make the mesh using two or more different types of fibers depending on the particular application and desired physical characteristics of the implant. A mesh that has both bioabsorbable and non-bioabsorbable fibers can also be made.

The said multifunctional device (mesh) can be manufactured by employing known and conventional warp knitting apparatus and techniques, such as the tricot and Raschel knitting machines and procedures described in "Warp Knitting Production" by Dr. S. Raz, Melliand Textilberichte GmbH, Rohrbacher Str. 76, D-6900 Heidelberg, Germany (1987).

The fibers are melt-spun with the 2-screw extruder, where the polymer melt (e.g. at temperatures ranging from 200 degrees to 270 degrees Centigrade) are pressed through e.g. four round die holes having diameter of e.g. about 0.4 mm. After cooling, filaments are oriented freely in a two-step process at elevated temperature (e.g. 60 degrees to 140 degrees Centigrade) to a draw ratio of e.g. 4 to 8. The final filament diameter can be e.g. 50 micrometers. The filaments are knitted by using a weft-knitting machine, with the fabric having loop size ca. 1 mm.

Following knitting, the mesh is cleaned or scoured, and thereafter annealed to stabilize the fabric. For the latter operation, the mesh can be secured to a tenter frame which maintains the mesh at a predetermined width, the frame then being passed through an elongated heating zone. Following heat setting, the mesh is cut to size, packaged and sterilized.

The mesh can be cut to any desired configuration, e.g. a square or rectangular shape of appropriate dimensions. An ultrasonic slitter may be employed to cut the mesh, various types of which are commercially available. Unlike the result one can obtain when cutting with a blade, i.e. frayed yarn ends, or when the yarn ends are heat-sealed; i.e. bead-like formations, cutting the mesh to the desired size.

A multifunctional mesh device can have two types of filaments, e.g. bioabsorbable and non-bioabsorbable. The pharmacological agent is included in the bioabsorbable filament. For example a non-bioabsorbable polypropylene monofilament exhibits good pliability. Depending on the material used to form the mesh, a mesh preferably has adequate flexibility. In addition, depending on the yarn - used to form the mesh, a mesh formed preferably has a sufficient burst strength.

In a preferred embodiment of the present invention, a mesh that has two different types of filaments is made of bioabsorbable polymer (filament type 1, e.g. PLGA 80/20) that degrades faster than the polymer used for making filament type 2, e.g. PLDLA 70/30 or PLDLA 96/4. Thus, the mesh degrades in two different rates, allowing tissues to develop and replace it in a more controlled and graduated fashion where such an effect is preferred/desired. The slower-to-degrade filaments remain for longer period of time and provide for longer time the desired strength, shape, and protection to treated tissue or tissue defect or damage, while the regenerating tissue gains its strength and density gradually reducing the sudden exposure to load and force, such as in hernia repair and thus, minimising the risks of recurrence, e.g. of hernia. As the filaments degrade away, simultaneously, repair tissue grows to cover the implant parts and detaching from the implant appear particles on top and in between its components (Länsman et al. 2002), starting from the surface of the implant or its components. The implant resorbs eventually, and is replaced with new repair tissue. Resorption products disappear from the body via metabolic routes. In the end, the tissue defect is filled in by the patient's own regenerated and/or repaired tissue. The pharmacological agent can be included only in one of the filament types or in both filament types.

PLGA 80/20 or PLDLA 70/30 pellets are introduced into an extruder and the extruded material then is drawn to predetermined draw ratios. According to the present invention, the antibacterial agent (antibiotic) is first dispersed throughout the polymer resin in powder form. The resin may be introduced directly into the extruder in powder form, or the resin containing the antibacterial agent may be formed into pellets, which then are introduced into the extruder. The extruded filament, containing pharmacological agent, for example, but not limited to, antibacterial agent, may be used then to prepare multifunctional surgical devices according to the present invention. Other pharmacological agents or biomolecules that may enhance the function or the multifunctionality of the device may be used employing similar manufacturing techniques.

In view of the process conditions to which the pharmacological agent, e.g. antibiotic may be exposed to unwanted effects, during preparation of the polymer resin, extrusion of the filament and/or yarn and/or rod and/or granules and subsequent production of the multifunctional surgical device itself. The pharmacological agent, in this case, the antibiotic must be selected to withstand being exposed to such manufacturing conditions without undergoing significant loss of its pharmacological, e.g. antibacterial properties. The agent must also be dispersible in the polymer material used to prepare the extruded filaments to make subsequently sutures and meshes, etc.

Antibacterial agents utilized in the present invention may be selected, e.g. from the group synthetic fluoroquinolones. An example of an antibacterial agent can be, e.g. ciprofloxacin (See example 1 below).

Filaments used to prepare multifunctional devices of the present invention are prepared by extrusion of a polymer material. During manufacture of the device, some of the pharmacological agent, which in the preferred example of the present invention is antibiotic, is typically lost due to processing procedure and conditions and/or sterilization but not after storage. The amount of the pharmacological agent, e.g. antibiotic used in preparing the polymer resin is determined based on the amount of agent actually required to be present in the multifunctional device to provide its intended multifunctionality, combined surgical management and medical therapy. In the preferred embodiment of the present invention, the said pharmacological agent is antibiotic whose containment in the device and subsequent release confers resistance to bacterial attachment, growth and biofilm formation on the surface of the device, taking into account the potential loss of agent due to processing procedures and conditions. The composite material comprises pharmacological agent, e.g. an antibiotic substantially dispersed therethrough in amounts effective to produce extruded filaments that, in turn, are useful in preparing multifunctional surgical devices that can have additional function such as, but not limited to, antibacterial function in addition to their primary function for tissue repair and management or tissue engineering, substantially preventing *in situ* bacterial attachment, growth or biofilm formation on the surface thereof, etc.

The compounding of the pharmacological agent, e.g. antibiotic into the polymer is performed in several steps. The polymer material and the pharmacological agent are fed through a compounding device. Feeding can be accomplished by force, gravity or starve feed systems. The extruder heats and mixes the materials until they are uniformly blended. A twin-screw extruder produces good mixing by forcing the melt back and forth from one screw to the other, thus breaking up flow patterns. The melted compound exits the extruder in strands that are quenched (cooled/crystallized) by water or air and cut into pellets. Those pellets can be then fed into a second extruder where the actual fiber will be manufactured.

Accordingly, in such an application example, the composite material must comprise a minimum amount of the antibiotic such that, upon manufacture of the multifunctional surgical device, the said device will be effective to inhibit *in situ,* bacterial attachment, growth, and biofilm formation on its surface. While such multifunctional devices may have trace amounts of bacteria present thereon, the levels of bacteria attached *in situ* will be significantly and will be sufficiently low so as not to lead to (persistent) infection or require addition of antibacterial agents applied to the surface of the device in order to avoid infection, e.g. surface coatings containing anti-microbial agents and significantly lower than traditionally-used implants. The maximum amount of the pharmacological agent that can be used will be limited by the desired properties of specific multifunctional surgical devices. For example, excessive amounts of the agent may over-plasticize or otherwise detrimentally affect the properties of the multifunctional surgical device disclosed in the present invention. Accordingly, the composite material may comprise from about 0.01 to about 50 weight percent of the antibacterial agent, preferably 0.1-30%, based on the total weight of the polymer and the used antibacterial agent. Most preferably, the compound material will comprise from about 1-10 % weight percent of the antibacterial agent. Once having the benefit of the present invention, one skilled in the art will readily ascertain the effective minimum and maximum amounts of the pharmacological agent, e.g. antibiotic.

Self-reinforced multifunctional devices of the present invention are especially important in the sense the can be used in prophylaxis and/or treatment of infection, because the said self-reinforced devices have better strength and strength retention properties than corresponding non-self-reinforced counterparts (Kangas, Mäkelä et al.). Self-reinforcing can render the devices of the present invention more reliable, and it allows for their wider clinical application.

One type of the multifunctional devices of the present invention can comprise alternating types of fibres (in their multifilamentous structure) that contain different pharmacological agents or biomolecules, e.g. one type of fibre can be an antibiotic-releasing whereas the other growth factor or analgesic or local anesthetic releasing, depending on the clinical indication and site of implantation. The latter can be advantage in more painful operations in their postoperative course, e.g. hernia operations, or in cases of chronic pain such as in cancer patients or in situation should pain occur, it is difficult to manage such may occur in thoracotomy procedures.

The fibers or filaments where the pharmacological agent is dispersed can be either hollow or solid.

The multifunctional device of the present invention can be a part of hybrid device where the said hybrid device contains also non-bioabsorbable fibres or sutures such as may needed for permanent or prolonged support as in the treatment of hernia using techniques such as Lechtenstein's technique. Hernia mesh can be made to have alternating multifunctional bioabsorbable sutures or fibers and non-bioabsorbable ones made into one hybrid devices prepared as mesh or fabric.

Following are examples that illustrate further but do not limit the present invention. Modifications of the present invention can be made without departing from the principles of the said invention.

### EXAMPLES

The following examples illustrate the multifunctional, characteristics of the surgical devices disclosed in the present invention and its preferred embodiment, e.g. characteristics of a PLDLA 70/30, or PLGA 80/20 material comprising no antibacterial agent, i.e. a non-multifunctional device, compared to a corresponding multifunctional device formed from a material comprising about 8% by weight of the active pharmacological agent.

### EXAMPLE 1: Ciprofloxacin-releasing SR-PLGA 80/20 suture

The used additive and pharmacologically active agent, ciprofloxacin, an antibiotic, was melt-blended in the matrix. The additive agent retained its solid particulate form in the melt-processing temperature of the matrix because of its melting or decomposition temperature higher than the melting temperature of the matrix. The blend was melt-spun into filament, which was drawn. The obtained product and a reference product were subjected to mechanical tests. The product according to the invention showed increased flexibility compared with reference product of the same composition and prepared in the same way but without the inclusion of the additive agent.

The following example is of a pharmacological agents of other category, a non-steroidal anti-inflammatory drug.

### EXAMPLE 2: Diclofenac-releasing monofilaments

Commercial PuraSorb^{®}PLG (Purac Biochem bv., Gorinchem, Netherlands) was used as basic polymer material. Diclofenac sodium (DS) was compounded into PLGA 80U20G matrix with a small twin-screw extruder. The loading of DS was 4 wt-%. Reference PLGA 80U20G and diclofenac sodium containing PLGA 80L/20G monofilaments were melt-spun by small laboratory extruder. The filaments were oriented in-line during melt-spinning. Drawing ratio was 4.8.

The tensile test for non-sterile monofilaments was done with Instron 4411 universal testing machine (Instron Ltd., Hiwh Wycombe, England). The test was performed using pneumatic jaws in distance of 50 mm. The tensile speed was 30 mm/min. The test was performed using the force cell of 500 N. Five parallel samples were taken about 50 cm distance from each other.

### Release on diclofenac in vitro has been studied with the sample:

| Sample | Sample [mg] | Buffer [ml] |
|---|---|---|
| PLGA 80U20G-DS4 | 200 | 10 |

In all parallel series buffer was (KH₂PO₄ and NaOH) adjusted in pH 7,4 ± 0,02. The samples were incubated at 37°C and buffer was replaced after specific time periods. From replaced buffer released quantity of diclofenac from monofilaments was detected by spectrophotometer (UNICAM UV 540, Thermo Spectronic, Cambridge, UK). The maximum absorption was measured (λ = 275 nm) from the samples and the quantity of released analgesic was calculated according to the Beer-Lambert law. The number of parallel samples was two.

### RESULTS

### Mechanical properties and diclofenac release

The results of preliminary tensile test of nonsterile DS-monofilament are presented in table 2.

**Table 2. The results of tensile test for PLGA 80U20G-DS4, the sample diameters are 1: 0,363 mm, 2: 0,412 mm, 3: 0,400 mm, 4: 0,382 mm and 5: 0,462 mm.**

| Specimen number | Load at Max. Load [N] | Stress at Max. Load [MPa] | Strain at Max. Load [%] | Displacement at Max. Load [mm] | Young's Modulus [MPa] | Load at thresh Yield [MPa] | Stress at Yield [MPa] | Display at [nm] |
|---|---|---|---|---|---|---|---|---|
| 1 | 7.22 | 69.77 | 72.60 | 36.40 | 3344.90 | | 56.8 | |
| 2 | 6.03 | 45.21 | 52.03 | 26.06 | 2118.11 | | 22.8 | |
| 3 | 7.40 | 58.86 | 96.33 | 48.25 | 2568.14 | | 28.6 | |
| 4 | 7.52 | 65.59 | 85.20 | 42.67 | 2839.16 | | 36.3 | |
| 5 | 9.30 | 55.49 | 123.70 | 62.09 | 1864.74 | | 28.7 | |
| Mean | 7.49 | 58.98 | 85.97 | 43.10 | 2547.01 | | 34.7 | |
| S.D | 1.17 | 9.51 | 26.75 | 13.44 | 585.64 | | 13.3 | |

The results of PLGA-reference filament are presented in table 3 below.

**Table 3. The results of tensile test of reference PLGA 80U20G filaments, the sample diameters are 1: 0,26 mm, 2: 0,25 mm, 3: 0,26 mm, 4: 0,25 mm and 5: 0,25 mm.**

| Specimen number | Load at Max. Load [N] | Stress at Max. Load [MPa] | Strain at Max. Load [%] | Displacement at Max. Load [mm] | Modulus (Aut Youg) [MPa] | Load at Yield [MPa] | Stress at Yield [Mpa] | Display at [nm] |
|---|---|---|---|---|---|---|---|---|
| 1 | 16.81 | 316.62 | 24.16 | 12.05 | 5146.82 | | 316.5 | |
| 2 | 16.08 | 327.58 | 26.46 | 13.25 | 5613.80 | | 327.6 | |
| 3 | 17.70 | 333.38 | 27.99 | 14.01 | 5370.88 | | 332.2 | |
| 4 | 16.86 | 343.47 | 25.46 | 12.76 | 5715.23 | | 341.8 | |
| 5 | 18.23 | 371.38 | 28.20 | 14.12 | 5966.01 | | - | |
| Mean | 17.14 | 338.48 | 26.45 | 13.24 | 5562.55 | | 329.53 | |
| S.D | 0.84 | 20.80 | 1.71 | 0.87 | 315.55 | | 10.50 | |

The release of diclofenac is shown below:

| Time [h] | Abs. 1 | Abs. 2 |
|---|---|---|
| 6 | 0.6593 | 0.639 |
| 18 | 0.038 | 0.0615 |
| 48 | 0.0176 | 0.0355 |
| 72 | 0.0169 | 0.03 |

### Conclusions

The addition of diclofenac increases tensile elongation of monofilaments and decreases Young's modulus of monofilaments. Diclofenac-containing monofilaments are more elastic compared to reference PLGA 80L/20G monofilaments without DS.

It can be shown that release properties of the device can also be influenced by inducing voids around the pharmacological agent particles. With self-reinforced (oriented) rods of PLGA 80/20 containing diclofenac sodium, increased amounts of released agent during the first weeks have been detected *in vitro.*

The surgical bioabsorbable device is not restricted to a special form, but it can be also other than a suture, fiber, thread, cord, wire or their derivative. It can be in a form which can be oriented to create cavities, for example plate, membrane, rod, or tube, which all may have changed physical and release profile properties resulting from the said mixing-orientation process.

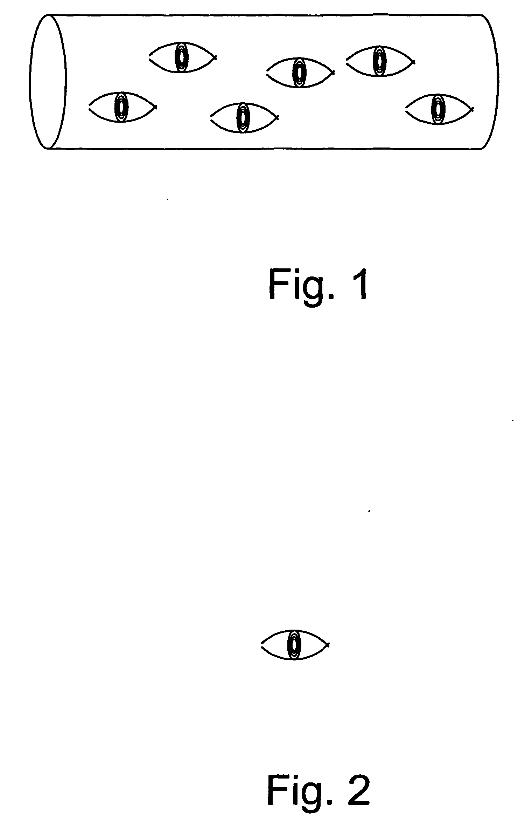

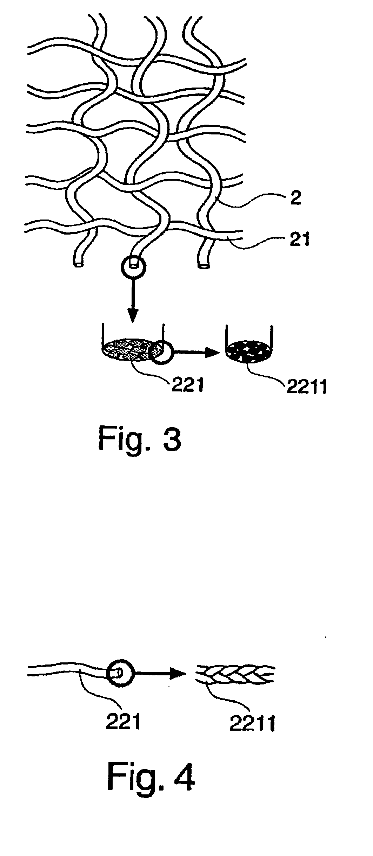

In the enclosed drawings, Fig. 1 illustrates the particles of the pharmacological agent in cavities within the matrix, as a result of the mechanical solid-state processing of the mixture, Fig. 2 showing a single cavity. The particles can also be in groups or clusters in cavities. Fig. 3 shows a mesh where multifilaments 2 and 21 are woven to an open fabric. As Fig. 3 shows, a single multifilament 2 may consist of several filaments 221, which may be multifilaments consisting of several monofilaments 2211. Fig. 4 shows a braid 221, which has two monofilaments 2211 braided together.

## Claims

1. A multifunctional synthetic bioabsorbable device comprising a synthetic bioabsorbable polymeric matrix and additive agent in the form of pharmacological agent, **characterized in that** cavities induced around the solid particles of the pharmacological agent dispersed in said matrix exist in said matrix as a result of orientation and mechanical solid-state processing of the mixture of the matrix and said particles, wherein the pharmacological agent retains its solid particulate form in the melt-processing temperature of the matrix.

2. A multifunctional synthetic bioabsorbable device of claim 1 especially for wound closure, **characterized in that** it has reduced Young's modulus and increased elasticity because of the cavitated spindle-shaped or oval-shaped porous structure resulting from the processing of said mixture.

3. The multifunctional device of claim 1, **characterized in that** the device is a suture, fiber, thread, cord, wire, or any derivative of these.

4. The multifunctional device of claim 3, **characterized in that** the device is a mesh.

5. The multifunctional device of claim 4, **characterized in that** the device is a mesh comprising fibers of differing bioabsorbable properties, such as
- absorbable multifunctional fibres of claim 1 together with non-bioabsorbable fibres, or
- fibres of differing bioabsorbtion rates .

6. The multifunctional device of any of the preceding claims, **characterized in that** the additive agent is an antibiotic.

7. The multifunctional device of any of the preceding claims, **characterized in that** said additive agent comprises 0.01 to 50 wt-% preferably 1-10 wt-% of the weight of the said multifunctional device.

8. The multifunctional device of claim 3, 4, 5, 6 or 7, **characterized in that** the said multifunctional device is monofilamentous in its structure.

9. The multifunctional device of claim 3, 4, 5, 6 or 7, **characterized in that** the said multifunctional device is multifilamentous in its structure.

10. The multifunctional device of any of the preceding claims, **characterized in that** the said multifunctional device has a drug releasing function effective to inhibit bacterial attachment and biofilm formation.

11. The multifunctional device of any of the preceding claims, **characterized in that** it is made by melt or solution processing technique, such as compounding with twin-screw extruder, and subsequent processing method, such as fibre spinning.

## Patentansprüche

1. Multifunktionelle synthetische bioabsorbierbare Vorrichtung umfassend eine synthetische bioabsorbierbare Polymermatrix und ein Zusatzmittel in Form eines pharmakologischen Wirkstoffs, **dadurch gekennzeichnet, dass** Hohlräume, die rings um die Feststoffteilchen des in der Matrix dispergierten pharmakologischen Wirkstoffs gebildet sind, in der Matrix als Folge der Ausrichtung und mechanischen Festkörperverarbeitung der Mischung aus Matrix und Teilchen vorliegen, wobei der pharmakologische Wirkstoff seine Feststoffform bei der Schmelzverarbeitungstemperatur der Matrix beibehält.

2. Multifunktionelle synthetische bioabsorbierbare Vorrichtung nach Anspruch 1 insbesondere zum Wundverschluss, **dadurch gekennzeichnet, dass** sie wegen der sich aus der Verarbeitung der Mischung ergebenden spindelförmigen oder ovalen porösen Struktur mit Hohlräumen einen reduzierten Youngschen Modul und eine erhöhte Elastizität aufweist.

3. Multifunktionelle Vorrichtung nach Anspruch 1, **dadurch gekennzeichnet, dass** die Vorrichtung ein Nahtmaterial, eine Faser, ein Faden, eine Schnur, ein Draht oder irgendein Derivat dieser ist.

4. Multifunktionelle Vorrichtung nach Anspruch 3, **dadurch gekennzeichnet, dass** die Vorrichtung ein Netz ist.

5. Multifunktionelle Vorrichtung nach Anspruch 4, **dadurch gekennzeichnet, dass** die Vorrichtung ein Netz umfassend Fasern mit unterschiedlichen bioabsorbierbaren Eigenschaften ist, beispielsweise
- absorbierbare multifunktionelle Fasern nach Anspruch 1 zusammen mit nicht-bioabsorbierbaren Fasern, oder
- Fasern mit unterschiedlichen Bioabsorptionsraten.

6. Multifunktionelle Vorrichtung nach irgendeinem der vorangehenden Ansprüche, **dadurch gekennzeichnet, dass** das Zusatzmittel ein Antibiotikum ist.

7. Multifunktionelle Vorrichtung nach irgendeinem der vorangehenden Ansprüche, **dadurch gekennzeichnet, dass** das Zusatzmittel 0,01 bis 50 Gew.-%, vorzugsweise 1-10 Gew.-% des Gewichts der multifunktionellen Vorrichtung umfasst.

8. Multifunktionelle Vorrichtung nach Anspruch 3, 4, 5, 6 oder 7, **dadurch gekennzeichnet, dass** die multifunktionelle Vorrichtung in ihrer Struktur monofil ist.

9. Multifunktionelle Vorrichtung nach Anspruch 3, 4, 5, 6 oder 7, **dadurch gekennzeichnet, dass** die multifunktionelle Vorrichtung in ihrer Struktur multifil ist.

10. Multifunktionelle Vorrichtung nach irgendeinem der vorangehenden Ansprüche, **dadurch gekennzeichnet, dass** die multifunktionelle Vorrichtung eine Arzneimittel freisetzende Funktion aufweist, die wirksam eine bakterielle Anhaftung und Biofilmbildung hemmt.

11. Multifunktionelle Vorrichtung nach irgendeinem der vorangehenden Ansprüche, **dadurch gekennzeichnet, dass** sie durch ein Schmelz- oder Lösungsverarbeitungsverfahren wie beispielsweise Mischen mit einem Doppelschneckenextruder und ein nachfolgendes Verarbeitungsverfahren wie beispielsweise Faserspinnen hergestellt ist.

## Revendications

1. Dispositif bioabsorbable synthétique multifonctionnel comprenant une matrice polymère bioabsorbable synthétique et un additif sous la forme d'un agent pharmacologique, **caractérisé en ce que** des cavités induites autour des particules solides de l'agent pharmacologique dispersé dans ladite matrice sont obtenues dans ladite matrice suite à l'orientation et au traitement mécanique à l'état solide du mélange de la matrice et lesdites particules, l'agent pharmacologique conservant sa forme particulaire solide à la température de traitement en fusion de la matrice.

2. Dispositif bioabsorbable synthétique multifonctionnel selon la revendication 1, en particulier pour refermer des blessures, **caractérisé en ce qu'**il possède un module de Young réduit et une plus grande élasticité, grâce à la structure poreuse comportant des cavités, en forme de fuseaux ou de forme ovale, qui résulte du traitement dudit mélange.

3. Dispositif multifonctionnel selon la revendication 1, **caractérisé en ce que** le dispositif est un fil de suture, une fibre, un fil, un cordon, un fil métallique ou l'un quelconque de leurs dérivés.

4. Dispositif multifonctionnel selon la revendication 3, **caractérisé en ce que** le dispositif est une maille.

5. Dispositif multifonctionnel selon la revendication 4, **caractérisé en ce que** le dispositif est une maille comprenant des fibres possédant des propriétés bioabsorbables différentes, telles que :
- des fibres multifonctionnelles absorbables selon la revendication 1, conjointement avec des fibres non-bioabsorbables ; ou
- des fibres possédant des degrés différents de bioabsorption.

6. Dispositif multifonctionnel selon l'une quelconque des revendications précédentes, **caractérisé en ce que** l'additif est un antibiotique.

7. Dispositif multifonctionnel selon l'une quelconque des revendications précédentes, **caractérisé en ce que** ledit additif comprend de 0,01 à 50 % en poids, de préférence de 1 à 10 % en poids du poids dudit dispositif multifonctionnel.

8. Dispositif multifonctionnel selon la revendication 3, 4, 5, 6 ou 7, **caractérisé en ce que** le dispositif multifonctionnel est de structure monofilamenteuse.

9. Dispositif multifonctionnel selon la revendication 3, 4, 5, 6 ou 7, **caractérisé en ce que** le dispositif multifonctionnel est de structure multifilamenteuse.

10. Dispositif multifonctionnel selon l'une quelconque des revendications précédentes, **caractérisé en ce que** ledit dispositif multifonctionnel possède une fonction de libération de médicament efficace pour inhiber la fixation bactérienne et la formation d'un biofilm.

11. Dispositif multifonctionnel selon l'une quelconque des revendications précédentes, **caractérisé en ce qu'**il est obtenu par une technique de traitement en fusion ou en solution, comme par exemple le mélangeage avec une extrudeuse à double vis sans fin et un procédé de traitement ultérieur tel que le filage de fibres.
